# EUROPEAN PATENT SPECIFICATION

(11) **EP 1 696 852 B1**
(45) Date of publication and mention of the grant of the patent: **30.10.2013**
(21) Application number: 04809135.9
(22) Date of filing: 22.12.2004
(51) Int. Cl.: A61F 13/49, A61F 13/493

(54) **SHAPE-ADAPTED ABSORBENT ARTICLE WITH IMPROVED ABSORPTION CAPACITY**
FORMADAPTIERTER SAUGFÄHIGER ARTIKEL MIT VERBESSERTER ABSORPTIONSEINGESCHAFT
ARTICLE ABSORBANT A ADAPTATION DE FORME AYANT UNE CAPACITE D'ABSORPTION AMELIOREE

(30) Priority: 23.12.2003 SE 0303521
(43) Date of publication of application: 06.09.2006
(73) Proprietor: SCA Hygiene Products AB, 405 03 Göteborg (SE)
(72) Inventor: ASP, Fredrik, S-439 35 Onsala (SE); CARLEN, Henrik, S-412 58 Göteborg (SE); HANSSON, Morgan, S-421 35 Västra Frölunda (SE)
(74) Representative: Romare, Laila Anette
(86) International application number: PCT/SE2004/001962
(87) International publication number: WO 2005/060892

(56) References cited:
- WO-A1-99/59907
- US-A- 4 029 100
- US-A- 5 713 883
- US-A- 6 090 090
- US-A- 6 114 597
- US-A1- 2002 165 512
- US-A1- 2003 125 700
- US-A1- 2003 135 177
- US-B1- 6 613 955

## Description

### TECHNICAL FIELD

The invention relates to an absorbent article with a longitudinal direction and a transverse direction and comprising an absorption body which is enclosed in a cover and comprises at least one absorption layer, the cover having a liquid-permeable surface and a liquid-impermeable surface. The invention also relates to a method for manufacturing an absorbent article as described above.

### BACKGROUND ART

Absorbent articles such as diapers, sanitary towels and incontinence shields are intended to catch and absorb body discharges of various kinds. Depending on the area of use and the quantity and nature of body discharge the article is to be capable of absorbing, there are of course articles of different shape and size. Diapers for heavily incontinent adults, for example, are of course considerably larger and have greater absorption capacity than diapers for infants. Furthermore, in addition to requirements for sufficient absorption capacity and leakage security, there are also requirements for the articles to be comfortable to wear. For adult wearers, it is moreover important that the articles are discreet and can be worn without being noticed under normal clothes.

The absorbent articles therefore have to be designed with sufficient absorption capacity to be capable of absorbing the said body discharge but still have a good fit so that they are pleasant and flexible to wear and so that they follow the body of the wearer closely and prevent leakage. One difficulty in designing an absorbent article intended to be worn in the crotch area of a wearer is that the space between the legs of the wearer is limited. This means that, in order to be comfortable to wear, the article must be narrowest within the area where the majority of the discharge from the wearer will meet the article. There is therefore an obvious risk that that area of the article which is affected first by body discharge will be oversaturated and will not be capable of absorbing any more when the next evacuation takes place. This means that the risk of leakage is already great when the article has absorbed relatively small quantities of body discharge in relation to its total capacity. In order to prevent leakage past the side edges of an absorbent article of this kind, it is therefore common to provide the article with some type of edge barrier. Such edge barriers are in most cases elastic and form raised physical obstacles to the passage of discharges. On diapers and incontinence shields of the kind which is worn like a pair of absorbent underpants, it is common to arrange elastic elements which fit tightly around the legs of the wearer and in this way hold the edges of the article in sealing contact with the legs.

On articles such as diapers and incontinence shields for heavily incontinent people, elastic leg bands and raised barriers are usually combined with the article being designed with a relatively wide crotch portion so as to bring about sufficient absorption capacity within the wetting area of the article. During use, such a crotch portion will be folded together between the legs of the wearer or will hang down as a liquid-collecting bag between the legs. Such a design creates random channels, which can give rise to leakage, is not especially comfortable or discreet and moreover functions poorly when the wearer sits down. For sanitary towels and other absorbent articles where the requirement for discretion during use is particularly high, such an unwieldy and inconvenient construction is not at all acceptable. Articles such as sanitary towels and incontinence shields for mildly incontinent people are moreover rather small and not self-supporting but are fastened inside a pair of ordinary briefs which hold the article in contact with the body of the wearer during use. A construction with a crotch portion which hangs down or is folded together cannot therefore be used for this category of article.

In order to manufacture shape-adapted and size-adapted absorbent articles with varying absorption capacity, a method is also required in which most of the individual manufacturing steps have to be adapted in order to provide articles of suitable shape and size. In this connection, it is also usually necessary to exchange most of the components required for the manufacturing method in order for it to be possible to obtain the said shape-adapted and size-adapted articles. In manufacturing, resetting equipment represents an operation which on the one hand results in a technically more complicated process and on the other hand adds extra costs in the form of machine components and costs for resetting times. As cost is a critical factor in the manufacture of disposable absorbent articles, such as shape-adapted absorbent articles, it is essential that absorbent articles of this kind can be manufactured in a simple and effective way so that the production cost and, at the next stage, the selling cost can be kept low.

As is evident from the above, there is a need for an absorbent article which has a good fit, high leakage security, high absorption capacity in the area which is affected first by body discharge, and which is discreet and comfortable to wear. It is also desirable to be able to provide an absorbent article which has such a good fit that special shaping elements or leakage barriers, for example in the form of elastic elements, can be avoided. There is moreover a need for a simple and cost-effective method for manufacturing the said absorbent article.

### DISCLOSURE OF INVENTION

By means of the present invention, an article has been produced with a longitudinal direction and a transverse direction and comprising an absorption body enclosed in a cover, the cover having a liquid-permeable surface and a liquid-impermeable surface, and the absorption body comprising at least one absorption layer, which article has a good fit, high leakage security, high absorption capacity, and high discretion and comfort.

An article made according to the invention is characterized mainly in that the absorption body comprises a first absorption part which is separate and displaced in the longitudinal direction from a second absorption part, which first absorption part has a tongue-shaped portion intended to be arranged in between two legs extending essentially in the longitudinal direction on the second absorption part so that fold indications are formed in the absorption body between the tongue-shaped portion of the first absorption part and at least each leg of the second absorption part.

By folding the first and second absorption parts, respectively, in relation to one another along the fold indications formed between the first and second absorption parts, a curvature of the absorption body is brought about in at least the area where the tongue-shaped portion of the first absorption part lies adjacent to each leg of the second absorption part. The curvature is therefore formed by virtue of the fact that, when folding takes place in the said fold indications, each leg of the second absorption part is folded in relation to the tongue-shaped portion of the first absorption part so that a bowl-shape is formed with the legs constituting sides of the bowl. In this way, the curved absorption layer is highly suitable as a component in an absorbent article, for example a diaper, a sanitary towel, an incontinence shield or the like, as the curved shape essentially follows the curvature of the body of the wearer.

Absorbent articles of this kind usually have two end portions and a crotch portion which is located between the end portions and has a smaller extent in the transverse direction than the end portions. In this connection, it is especially advantageous that the fold indications in the absorption body are arranged at least mainly in the crotch portion. The fold indications are suitably formed in the absorption body between the tongue-shaped portion of the first absorption part and at least each leg of the second absorption part on both sides of a centre line extending along the article in the longitudinal direction. In this connection, the fold indications extending in the longitudinal direction are suitably arranged at a distance from one another in the transverse direction which essentially corresponds to the width of the crotch area of a wearer.

In order to obtain an absorbent article of the kind referred to in the introduction, it is suitable that the two legs extending in the longitudinal direction on the second absorption part have a length, s, of 3-30 cm, preferably 5-25 cm, and most preferably 7-20 cm and/or that the tongue-shaped portion of the first absorption part has a length, t, of 3-30 cm, preferably 5-25 cm, and most preferably 7-20 cm.

For diapers for children, it is suitable that each fold indication extending in the longitudinal direction has a length of 3-20 centimetres, preferably about 15 centimetres, and that the spacing in the transverse direction between the fold indications extending in the longitudinal direction has a width of 1-5 centimetres, preferably about 3 centimetres. For diapers and other types of body-enclosing incontinence shields for adults, it is suitable that each fold indication extending in the longitudinal direction has a length of 3-30 centimetres, preferably about 20 centimetres, and that the spacing in the transverse direction between the fold indications extending in the longitudinal direction has a width of 1-7 centimetres, preferably about 4 centimetres. In this connection, the length, I, extending in the longitudinal direction, of a fold indication means the distance between the point which essentially corresponds to the outermost tip of a leg and the termination point of the leg. In this connection, the termination point of a leg is essentially located where the inner edge of a leg starts to merge with the U-shaped inner edge of the second absorbent part, which U-shaped inner edge lies between the two termination points. The width of the folded portions means the distance in the transverse direction between the fold edges formed at the respective fold indications. For sanitary towels, or incontinence shields for mildly incontinent people, the fold indications suitably have a length between the end points of 5-20 centimetres, preferably about 10 centimetres, and the folded portions have a width of 1-5 centimetres, preferably 2.5 centimetres.

In order to obtain a more accentuated fold indication, there can be a distance of 1-20 mm, preferably 2-15 mm, and most preferably 3-10 mm, between the tongue-shaped portion of the first absorption part and each leg of the second absorption part, at least at the outer parts of the legs. Here, the outer parts of the legs mean the whole of the length, I, extending in the longitudinal direction, of the fold indication, preferably half the length, I, seen from the outermost tip of the leg concerned, and most preferably a third of the length, I, seen from the outermost tip of the leg concerned. In addition to the advantage that it will thus be possible for the fold indication to allow a more marked fold between the parts located on each side of the fold indication, a space which is free from absorbent material will also be provided between the first absorption part and each leg of the second absorption part. This free space is advantageous in particular when a large quantity of liquid is discharged in a short time. Liquid can then be caught rapidly in this free space, to be subsequently absorbed by surrounding material. The advantages of this embodiment become even more apparent after a first wetting, that is to say on subsequent wettings when further discharged liquid has to be distributed in the material of the core parts, which is already wet and may have swollen owing to liquid absorption. However, the tongue-shaped portion of the first absorption part and each leg of the second absorption part can also, at least at the outer parts of the legs, advantageously be arranged so that the first absorption part and the second absorption part lie edge to edge with one another, a more shape-adapted product then being obtained.

As mentioned above, the first absorption part is designed with a tongue-shaped portion, it being possible for the tongue-shaped portion to have an essentially U-shaped outer edge. The second absorption part also extends from the tongue-shaped portion in the longitudinal direction and/or in the transverse direction so that a large free surface is formed, which large free surface is considerably larger than the surface of the tongue-shaped portion.

The second absorption part is designed so that its legs have an inner edge which comes in towards a central line in the longitudinal direction in the second absorption part so as thus to form an essentially U-shaped edge. The second absorption part also extends from its legs in the longitudinal direction and/or in the transverse direction so that a large free surface is formed. In this connection, the second absorption part has a tooth-like appearance where the legs may be said to correspond to the two roots of the tooth. The legs advantageously consist of the same absorption material as the free surface of the second absorption part. In order to increase the dimensional stability, however, the legs advantageously also comprise stiffening material in one embodiment.

When the first absorption part, according to the present embodiment, is adapted to the second absorption part so that fold indications are formed in the absorption body between the tongue-shaped portion of the first absorption part and at least each leg of the second absorption part, the fold indications will be most distinctly marked where the first absorption part and the second absorption part are arranged with a free space between them, or lie edge to edge. However, a less marked fold indication will also be formed in the situation where the first absorption part lies overlapping the second absorption part. The outer edge on the tongue-shaped portion of the first absorption part will then during use be pressed against the second absorption part and form a fold indication along which the second absorption part will be folded. The specifically mutually adapted shapes of the first absorption part and the second absorption part will consequently produce a curved shape which extends in both the transverse direction and the longitudinal direction over the crotch area. The invention as described above therefore provides a three-dimensional curved shape which can be adapted optimally to the crotch area of a wearer and which therefore brings about both a good fit and increased leakage security.

Depending on how accentuated the folding required is, the first absorption part and the second absorption part can be arranged in relation to one another so that a more marked fold is achieved. According to an advantageous embodiment of the invention, there is a distance between the U-shaped edge of the second absorption part and the tongue-shaped portion of the first absorption part of 0-20 mm, preferably 2-15 mm, and most preferably 3-10 mm, along the whole tongue-shaped portion. According to another embodiment, an inner edge of the legs of the second absorption part turns off in towards a longitudinal centre line so as thus essentially to correspond to and essentially to run edge to edge with the outer edge of the tongue-shaped portion of the first absorption part.

As mentioned above, the tongue-shaped portion of the first absorption part can overlap at least a part of the second absorption part. A less marked bowl shape of the absorption body can thus be obtained. However, there is also an advantage in an area with an overlap being formed. Such an overlap is then especially advantageous when increased absorption capacity is desired in parts of the absorbent article which will be subjected to great loading by body discharges. According to a preferred embodiment of the invention, the overlap is such that the tongue-shaped portion of the first absorption part overlaps a part of the second absorption part so that a double layer is formed in at least an area corresponding to the wetting zone of the absorbent article. Wetting zone means the zone which lies adjacent to the genitals of the wearer when the absorbent article is used.

According to one embodiment of the invention, the first absorption part is a rear absorption part located in a rear end portion of the article, which is intended to face backwards on the wearer during use, and the second absorption part is a front absorption part located in a front end portion of the article, which is intended to face forwards on the wearer during use. However, the first absorption part can be a front absorption part located in a front end portion of the article, and the second absorption part can be a rear absorption part located in a rear end portion of the article.

The front and rear absorption parts can advantageously comprise absorbent material of the same kind. The front absorption part can advantageously also be designed so that it comprises absorbent material specially intended for taking up urine and/or other viscous liquids such as menstrual fluids and discharges. The rear absorption part can also be designed so that it comprises absorbent material intended for taking up faeces and/or other viscous liquids such as menstrual fluids and discharges. A combination of a front absorption part intended mainly for urine absorption and a rear absorption part intended for taking up mainly faeces is especially advantageous when the front absorption part and the rear absorption part are separated from one another. Here, separated means that, while it is true that the parts can lie overlapping one another, they constitute two parts which are independent of one another. When the front and rear absorption parts comprise material intended specially for their respective take-up and when they are in addition advantageously not interconnected by any liquid-conducting material, the different body discharges can be kept separated and odour can be avoided. A mixture of urine and faecal matter is moreover particularly aggressive as far as action on the skin is concerned.

The front and rear absorption parts are advantageously movable in relation to one another to such an extent that a curved shape as described above can be formed. However, the first absorption part can be locked in a predetermined position in relation to the second absorption part by a stabilizing means, without the mobility between the two parts being lost. In a case where the stabilizing means is a stabilizing layer which is connected firmly to the first absorption part and the second absorption part, folds will still be formed in the said fold indications. The stabilizing layer can then be attached along the whole surface of the first absorption part and the second absorption part, respectively, or along parts thereof.

The stabilizing layer can constitute a distance layer between the first absorption part and the second absorption part, respectively, and comprise either the liquid-permeable surface of the cover or the liquid-impermeable surface of the cover. Alternatively, the stabilizing layer can consist of the liquid-permeable surface of the cover or of the liquid-impermeable surface of the cover, or both cover layers. When either or both of the cover layers are designed to stabilize the absorption body, each absorption part can be attached completely or partly to the layer(s) concerned depending on how great the mobility required is. The stabilizing layer can be attached by, for example, gluing or welding. The said stabilizing layer can consist of, for example, in addition to a liquid-permeable cover layer or a liquid-impermeable cover layer, a layer which in a finished absorbent article functions as a strengthening layer, a liquid-receiving layer or an additional absorption layer.

As mentioned above, a method for manufacturing shape-adapted absorbent articles with improved absorption capacity where the individual manufacturing steps can be adapted in order simply and effectively to provide articles of suitable shape and size is also particularly desirable.

A method for producing an absorption body made according to the invention for use in an absorbent article is characterized mainly in that the absorption body is produced by a first absorption part being shaped with a tongue-shaped portion and a second absorption part being shaped with two legs extending in the longitudinal direction. The first absorption part is positioned separately and displaced in the longitudinal direction in connection to the second absorption part so that the tongue-shaped portion of the first absorption part is arranged completely or partly in between the two legs extending essentially in the longitudinal direction on the second absorption part, fold indications then being formed in the absorption body between the tongue-shaped portion of the first absorption part and at least each leg of the second absorption part.

In manufacture, at least a part of the inner edge of the legs of the second absorption part can advantageously be positioned edge to edge with the outer edge of the tongue-shaped portion. The first absorption part can alternatively be applied to the second absorption part so that the tongue-shaped portion of the first absorption part overlaps at least a part of the second absorption part.

Normally, more than one size of absorbent article is manufactured on one and the same production machine. When the size of the article is to be changed, a number of machine components such as, for example, profile-cutting units, compression units or the like have to be exchanged in this connection. For an absorbent article according to the invention, however, a smaller number of machine components have to be exchanged. Above all, the mat-forming moulds of the machine, where the absorption bodies are produced, do not have to be exchanged, especially if the change in size is relatively small. When the size of the article is to be changed in a manufacturing machine which produces absorbent articles according to the invention, the first and second absorption parts are instead arranged overlapping one another to a greater or lesser extent or completely separated in the crotch portion of the absorbent article, the size then being changed. The time-consuming exchange of mat-forming moulds is then eliminated, making it possible for both the resetting time and the number of persons involved in the resetting process to be reduced significantly.

When the size of the article is changed, the intermixing of gel-forming material, what are known as superabsorbents, or the quantity of pulp fibres in the moulds can be increased/decreased by the material being pressed together to a greater or lesser extent in the moulds, it then being possible to adjust the absorption capacity of the article. The same advantage is also obtained in the manufacture of absorption bodies which are cut from a web-shaped absorption material. The absorption capacity can then be changed by selecting a web-shaped material comprising more or less superabsorbent material, and increasing or decreasing the weight per unit area of the web-shaped material.

The shape of the absorption body after positioning of the first absorption part and the second absorption part in relation to one another can also be fixed by joining the absorption body together with a stabilizing layer, for example by gluing or welding. In this connection, the shape of the absorption body can be fixed by activating a bonding means included in the absorption body, or by gluing or welding, to a layer included in the absorbent article, such as, for example, a stabilizing layer and/or one or both of the cover layers. According to one embodiment of the invention, the bonding means comprises thermoplastic fibres, and the shape of the absorption body is stabilized by heating and subsequent cooling of the absorption body.

### DESCRIPTION OF FIGURES

The invention will be described in greater detail below with reference to the illustrative embodiments shown in the accompanying drawings, in which connection:
- Figure 1: shows a diaper according to the invention;
- Figure 2a: shows a section along the line II-II through the diaper in Fig. 1 according to a first embodiment of the invention;
- Figure 2b: shows a section along the line II-II through a diaper in Fig. 1 in wearer position;
- Figure 3: shows an absorption body according to the invention, and
- Figure 4: shows another absorption body according to the invention.

### PREFERRED EMBODIMENTS

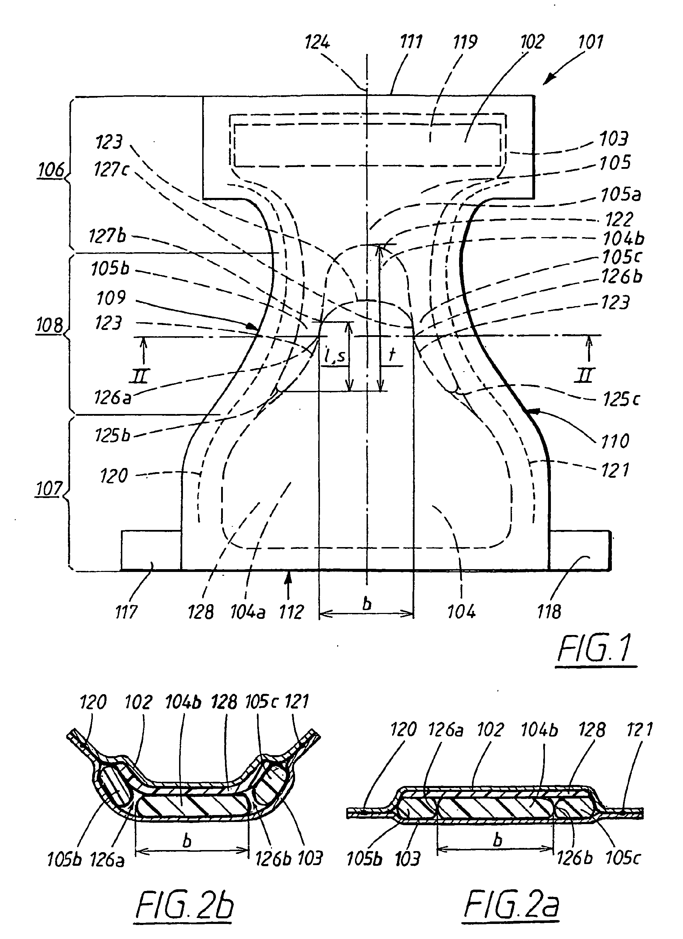

The diaper 101 shown in Fig. 1 comprises a first, liquid-permeable cover layer 102, a second, liquid-impermeable cover layer 103, and an absorption body which is arranged between the cover layers 102, 103 and comprises a first absorption part 104 and a second absorption part 105. The two cover layers 102, 103 have a greater extent in the plane than the absorption body and project beyond the absorption body around its whole periphery. The cover layers 102, 103 are interconnected within the projecting portions, for example by means of gluing or welding using heat or ultrasound.

The liquid-permeable cover layer 102 can consist of any material suitable for the purpose such as layers of nonwoven material, perforated plastic film, net material, tow (parallel continuous fibre threads) or the like. The cover layer 102 can of course also consist of a laminate of two or more layers of the same or different material. Within the scope of the invention, however, the liquid-permeable cover layer 102 does not have to be a separate component but can be an integrated part of the absorption body 104, 105. In such an embodiment, the liquid-permeable cover layer 102 is not necessarily included in the projecting edge around the absorption body 104. Examples of materials which can both be the cover layer 102 and form part of the absorption body 104 are foam, fibre wadding, nonwoven material or the like.

The liquid-impermeable cover layer 103 can consist of a liquid-impermeable plastic film, a nonwoven layer which has been coated with a liquid-blocking material, or another flexible material layer capable of withstanding liquid penetration. It can be an advantage, however, if the liquid-impermeable cover layer 103 has a certain breathability, that is to say it allows the passage of water vapour through the layer 103. Like the liquid-permeable cover layer 102, the liquid-impermeable cover layer 103 can be an integrated part of the first absorption part 104 and the second absorption part 105 of the absorption body and can consist of, for example, a liquid-impermeable skin-like surface on an absorbent foam material. If both the liquid-permeable and the liquid-impermeable cover layers 102, 103 constitute integrated parts of the absorption body, the first absorption part 104 and the second absorption part 105 included in the absorption body should be stabilized by means of a stabilizing layer.

The diaper 101 has an elongate shape, with wider front and rear portions 106, 107 and a narrower, intermediate crotch portion 108. In this connection, the front portion 106 is that part of the diaper 101 which is intended to face forwards on the wearer when the diaper is used, and the rear portion 107 is that part of the diaper which faces backwards on the wearer. The diaper 101 also has two longitudinal, inwardly curved side edges 109, 110, a front edge 111 and a rear edge 112.

The diaper 101 is of the kind which is fastened together when it is used, so that it surrounds the lower part of the abdomen of the wearer in a pant-like manner. For this purpose, a tape flap 117, 118 is arranged projecting from each side edge 109, 110, close to the rear edge 112 of the diaper. The tape flaps 117, 118 are intended to interact with a receiving area 119 arranged on the liquid-impermeable cover layer 103 on the front portion 106 of the diaper 101. Such a receiving area 119 suitably involves some form of strengthening of the liquid-impermeable cover layer 103, for example in the form of an extra plastic layer or a coating applied to the liquid-impermeable cover layer 103. It is of course alternatively conceivable to use other types of fastening devices for the diaper 101, such as buttons and buttonholes, hooks and eyes, press studs, hook-and-loop fasteners or the like. When hook-and-loop fasteners are used, these can suitably be arranged so that the male parts of the hook-and-loop fasteners are arranged as corresponding flaps 117, 118 and the female parts as a corresponding receiving area 119. Here, male parts mean the hook-shaped elements of the hook-and-loop fastener, and female parts mean the loop-shaped elements of the hook-and-loop fastener. However, the flaps 117, 118 can also consist of female parts and the receiving area 119 of male parts. Another alternative is for the diaper to be what is known as a pant diaper, which means that it is supplied to the wearer as continuous absorbent pants intended to be pulled over the legs. Pant diapers can also be openable. Another common diaper type is fastened together by means of a belt to which the diaper is attached or from which it is detachably suspended.

The diaper 101 is also provided with pretensionally arranged, essentially longitudinal elastic elements 120, 121, arranged along the side edges 109, 110 of the diaper. The elastic elements 120, 121 contribute to curving the diaper 101 according to the body of the wearer during use and at the same time constitute the leg elastic of the diaper. In this way, the elastic elements 120, 121 serve to hold the side edges 109, 110 of the diaper in sealing contact with the legs of the wearer so as to counteract gaps appearing between the diaper and the body of the wearer during use, through which body fluid can leak out of the diaper. The elastic elements are not necessary for the invention but can be omitted, as an absorption body according to the invention, and thus the diaper 101 also, has a very good fit and follows the body of the wearer closely even without elastic elements 120, 121.

The first absorption part 104 of the absorption body is arranged in an overlap over the second absorption part 105 so that a double layer of absorption material is formed in the area which will correspond to the wetting zone of the absorbent article. The first absorption part 104 is designed with a large free surface 104a and a tongue-shaped portion 104b which has an essentially U-shaped outer edge 122. The tongue-shaped portion 104b has a length, t, shown in the figure. The second absorption part 105 is designed with a large free surface 105a which runs out into two legs 105b, c. The absorption body is designed so that the free surface 104a of the first absorption part 104 will during use constitute part of the rear portion 107, and the free surface 105a of the second absorption part 105 will constitute part of the front portion 106. The tongue-shaped portion 104b and the legs 105b, c will constitute part of the crotch portion 108 lying between the front portion and the rear portion. The legs 105b, c have an inner edge 123 which runs in, in the direction of the free surface 105a of the second absorption part 105, the inner edge 123 also forming an essentially U-shaped edge. The two legs 105b, c extend essentially in the longitudinal direction along a longitudinal centre line 124 in the diaper 101 towards the outer tip 125b, c of the respective leg. Two fold indications 126a, b formed in essentially the longitudinal direction run at least between the outer tip 125b, c of the respective leg and the respective termination point 127b, c, which points are located essentially where the inner edge 123 of the legs starts to merge with the U-shaped edge of the second absorbent part. The distance between the outer tip 125b, c of each leg and the corresponding respective termination point 127b, c is indicated in Fig. 1 as a length I. In this embodiment, the length, I, corresponds essentially to the length, s, of the legs. Along the length, I, a part of the tongue-shaped portion 104b will lie edge to edge with the respective leg 105b, c, a marked fold indication part 126a, b being formed in this part. As the tongue-shaped portion 104b overlaps a part of the second absorption part 105, however, the fold indication 126a, b will also merge with a less marked portion. This less marked fold indication part 126a, b is formed by the outer edge 122 on the tongue-shaped portion 104b. The fold indications 126a, b will during use form a more marked fold along the length I, then merging with a less marked fold along the outer edge 122 of the tongue-shaped portion 104b as the latter is pressed against the second absorption part 105. As a whole, the fold indications 126a, b will during use make possible a curved, anatomically adapted shape fitted to the crotch portion of the wearer. The crotch portion of the absorbent article will during use have a width, b, which will essentially correspond to the distance in the transverse direction between the fold edges formed at the respective fold indications 126a, b.

By virtue of the overlap and the legs 105b, c which are bent during use, a thicker, more absorbent portion is obtained in that area of the diaper 101 which is expected to be wetted first by body fluid, at the same time as the crotch portion 108 can be made considerably narrower than is possible in conventional diapers. The extra quantity of absorption material is localized to a small, relatively narrow area of the crotch portion 108, which makes the diaper comfortable to wear in spite of the increased absorption capacity. The folded legs 105b, c also result in stabilization and stiffening of the crotch portion 108 and in this way counteract uncontrolled deformation and creasing, which also contributes to increased comfort and to the diaper being discreet to wear and leakproof.

The first absorption part 104 and the second absorption part 105 of the absorption body can be constructed from one or more layers of absorbent material, such as cellulose fluff pulp, tissue, absorbent foam etc. It is also common for the absorption body to contain superabsorbents, that is to say polymer materials which can absorb body fluid corresponding to several times their own weight while forming a hydrogel. Such superabsorbents are usually in the form of particles, but fibres, flakes, granulates and film are also found. The two parts 104, 105 of the absorption body can also comprise non-absorbent components such as stiffening elements, shaping elements, bonding means etc. Various types of liquid-receiving porous structures such as fibre waddings or the like can also be included in the diaper 101. In particular, the legs 105b, c and/or the outer edge 122 on the tongue-shaped portion 104b can advantageously comprise stiffening elements and/or shaping elements in order to increase the dimensional stability in the fold indication 126a, b.

The first and second absorption parts 104, 105 can advantageously comprise absorbent material of the same kind. Alternatively, the first and second absorption parts 104, 105 can comprise absorbent materials specially intended for taking up faeces and urine respectively, which is known within the art. The absorbent material can also be specifically adapted so as also to be capable of taking up viscous liquids such as menstrual fluids and discharges.

The diaper 101 also comprises a liquid-transfer layer 128, which is arranged between the liquid-permeable cover layer 102 and the two parts 104, 105 of the absorption body. In the illustrative embodiment shown, the liquid-transfer layer 128 is the same shape and size as the absorption body, which is of course not necessary for the invention. The liquid-transfer layer 128 can therefore be smaller or larger than the absorption body and have a shape different to that of the absorption body.

The liquid-transfer layer 128 between the liquid-permeable cover layer 102 and the absorption body suitably has higher porosity than the absorption body and has a good capacity for receiving liquid and for giving liquid up to the absorption body. The liquid-transfer layer 128 also constitutes a distance layer between liquid which has been absorbed in the absorption body and the body of the wearer and in this way prevents rewetting, that is to say body fluid already absorbed escaping back out through the liquid-permeable cover layer 102. In general, it is an advantage if the liquid-transfer layer 128 comprises a thermoplastic component which can be used in order to join the liquid-transfer layer 128 together with the liquid-permeable cover layer 102 and/or the absorption body. In this connection, the liquid-transfer layer 128 advantageously forms a stabilizing layer which fixes the first and second parts 104, 105 of the absorption body in relation to one another.

Fig. 2a shows a section along the line II-II through the diaper in Fig. 1. In Fig. 2, the liquid-transfer layer 128 extends over the first leg 105b of the first absorption part, the tongue-shaped portion 104b and the second leg 105c of the first absorption part. The shape of the absorption body is locked by joining the tongue-shaped portion 104b together with the liquid-transfer layer 128. The joining can be effected in any suitable way, for example by gluing, needling or welding or fusing thermoplastic components in the liquid-transfer layer 128 together. The liquid-transfer layer 128 positions only the legs 105b, c in a physical respect and is therefore not joined to said legs 105b, c. When the diaper 101 is folded along fold indications 126a, b, the legs 105b, c will therefore be freely movable in relation to the tongue-shaped portion 104b. It is also possible to fix the shape of the diaper by joining the absorption body together with the liquid-impermeable cover layer 103 or, if the liquid-transfer layer 128 is excluded, with the liquid-permeable cover layer 102.

An important advantage of the absorption body shown in Fig. 2a is that, when discharged liquid has been transported past the liquid-transfer layer 128, a certain quantity of liquid can easily pass through the liquid-admission channels for rapid transport of liquid into the absorption body formed by the fold indications 126a, b. The liquid can also be spread rapidly in the longitudinal direction of the diaper 101 in the fold indications 126a, b, then being absorbed by parts of the absorbent material which are more peripheral than the wetting point.

Fig. 2b shows a section along the line II-II through a diaper in Fig. 1 when the diaper is curved in accordance with the wearer position. Like the section of the diaper shown in Fig. 2a, a liquid-transfer layer 128 is shown, which extends over the first leg 105b of the first absorption part, the tongue-shaped portion 104b and the second leg 105c of the first absorption part. In a way corresponding to that described in Fig. 2a, the shape of the absorption body is locked by joining the tongue-shaped portion 104b together with the liquid-transfer layer 128. As can be seen from Fig. 2b, the liquid-transfer layer 128 positions only the legs 105b, c in a physical respect and is not joined to said legs 105b, c. When the diaper 101 is folded along fold indications 126a, b, the legs 105b, c are freely movable in relation to the tongue-shaped portion 104b. In this connection, the liquid-admission channels for rapid transport of liquid into the absorption body are formed in the fold indications 126a, b. The width of the liquid-admission channels formed can be adjusted by the liquid-transfer layer 128 being joined together with the legs 105b, c at a suitable distance from the tongue-shaped portion 104b. It is also possible, as described above, to fix the shape of the diaper by joining the absorption body together with the liquid-impermeable cover layer 103 or, if the liquid-transfer layer 128 is excluded, with the liquid-permeable cover layer 102.

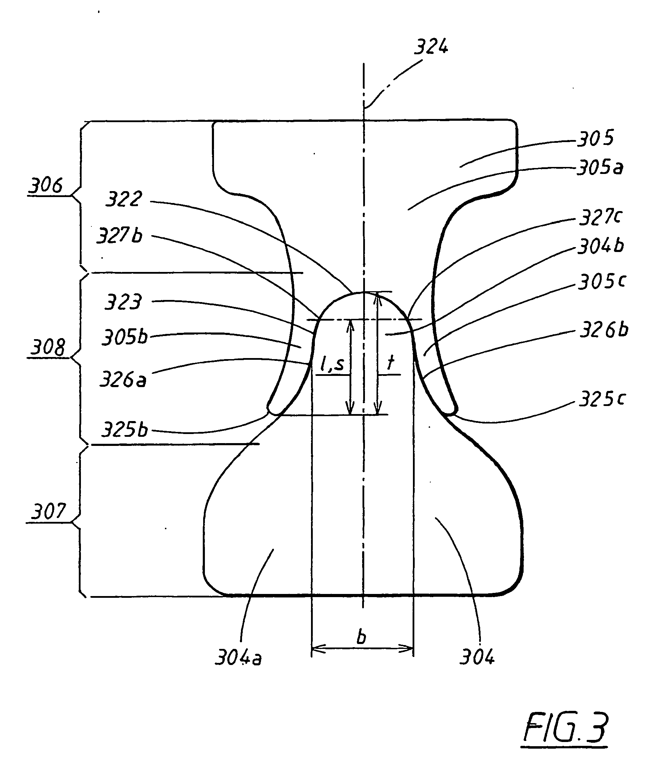

The absorption body shown in Fig. 3 has the same basic construction as the absorption body in Figs. 1 and 2 and therefore comprises a first absorption part 304 and a second absorption part 305. The first absorption part 304 has a large free surface 304a and a tongue-shaped portion 304b which has an essentially U-shaped outer edge 322. The tongue-shaped portion 304b has a length, t, shown in the figure. The second absorption part 305 has a large free surface 305a which runs out into two legs 305b, c. The absorption body is designed so that the free surface 304a of the first absorption part 304 will during use constitute part of the rear portion 307, and the free surface 305a of the second absorption part 305 will constitute part of the front portion 306. The tongue-shaped portion 304b and the legs 305b, c will constitute part of the crotch portion 308 lying between the front portion and the rear portion. In this connection, the front portion 306 is that part of the absorption body which is intended to face forwards on the wearer when the absorption body is used, and the rear portion 307 is that part of the absorption body which faces backwards on the wearer. The legs 305b, c have an inner edge 323 which runs in in the direction of the free surface 305a, the inner edge 323 having essentially a U-shape. The two legs 305b, c extend essentially in the longitudinal direction along a longitudinal centre line 324 towards the outer tip 325b, c of the respective leg. Two fold indications 326a, b formed in essentially the longitudinal direction run between the outer tip 325b, c of the respective leg and the respective termination point 327b, c, which points are located essentially where the inner edge 323 of the legs starts to merge with the curvature of the U-shaped part. The distance in the longitudinal direction between the outer tip 325b, c of each leg and the corresponding respective termination point 327b, c is indicated in the figure as a length I. This fold indication length, I, has a slightly longer extent in the longitudinal direction than the corresponding fold indication length, I, shown in Fig. 1, which gives rise to more accentuated folding of the absorbent body taking place over the crotch portion. In this embodiment as well, the length, I, corresponds essentially to the length, s, of the legs. In this embodiment, the whole outer edge 322 of the tongue-shaped portion 304b lies edge to edge with the whole inner edge 323, which means that the.fold indication will run along the whole of the U-shaped part of the tongue-shaped portion. More accentuated folding will then be obtained in both the transverse direction and the longitudinal direction of the absorption body, which leads to a more distinct bowl shape. A deeper bowl shape has been found to be advantageous inter alia as it retains liquid better even when the wearer is lying on a side.

The embodiment shown in Fig. 3 is also especially advantageous when the absorption body is adapted for separate take-up of urine and faeces. By virtue of the first and second absorption parts being more separated from one another than in the embodiment shown in Fig. 1, it is more difficult for body discharges to be transferred between the absorption parts. The odour which arises when different body discharges meet can thus be avoided.

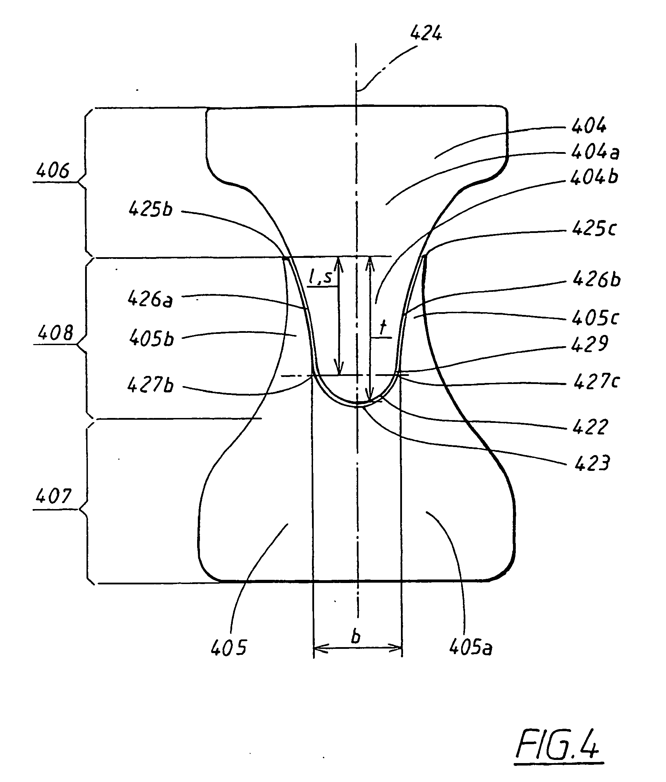

Fig. 4 shows an absorption body which, like the absorption bodies shown in Figs. 1 and 3, has a first absorption part 404 and a second absorption part 405. The first absorption part 404 also has a large free surface 404a and a tongue-shaped portion 404b which has an essentially U-shaped outer edge 422. The tongue-shaped portion 404b has a length, t, shown in the figure. The second absorption part 405 has a large free surface 405a which runs out into two legs 405b, c. In contrast to the absorption bodies shown in Figs. 1 and 3, the absorption body is in this embodiment positioned so that the free surface 404a of the first absorption part 404 will during use constitute part of the front portion 406, and the free surface 405a of the second absorption part 405 will constitute part of the rear portion 407. The tongue-shaped portion 404b and the legs 405b, c will constitute part of the crotch portion 408 lying between the front portion and the rear portion. In this connection, the front portion 406 is that part of the absorption body which is intended to face forwards on the wearer when the absorption body is used, and the rear portion 407 is that part of the absorption body which faces backwards on the wearer. The legs 405b, c also have an inner edge 423 which runs in in the direction of the free surface 405a, the inner edge 423 having essentially a U-shape. The two legs 405b, c extend essentially in the longitudinal direction along a longitudinal centre line 424 towards the outer tip 425b, c of the respective leg. Two fold indications 426a, b formed in essentially the longitudinal direction run between the outer tip 425b, c of the respective leg and the respective termination point 427b, c along a length, I, and also along the whole inner edge 423 along the U-shaped part. In this embodiment, the length, I, corresponds essentially to the length, s, of the legs. In this embodiment, the whole outer edge 422 of the tongue-shaped portion 404b is fixed at a distance of about 5 mm from the whole inner edge 423, which means that the fold indication will run along the whole of the U-shaped part of the tongue-shaped portion 404b. Even more accentuated folding will then be obtained in both the transverse direction and the longitudinal direction of the absorption body, which leads to a more distinct bowl shape.

One advantage of this embodiment is that a space 429 which is free from absorbent material is provided between the tongue-shaped portion 404b and each leg 405b, c. This free space 429 is advantageous as it functions as a channel in particular when a large quantity of liquid is discharged in a short time. As described above, liquid can then be caught rapidly in this free space, subsequently being absorbed by surrounding material. The advantages of this embodiment also become even more apparent after a first wetting, that is to say on subsequent wettings when further discharged liquid has to be distributed in the material of the core parts, which is already wet and may have swollen owing to liquid absorption. In the illustrative embodiment shown, the free space 429 is free from material. However, it is conceivable for the free space 429 to be completely or partly filled with a porous material, for example wadding or foam.

The embodiment shown in Fig. 4 is advantageous when the absorption body is adapted for separate take-up of urine and faeces. By virtue of the first and second absorption parts being completely separated from one another compared with the embodiments in Figs. 1 and 3, it is not possible for body discharges to be transferred between the absorption parts. The odour which arises when different body discharges meet can thus be avoided altogether. As the said mixture of urine and faecal matter is moreover particularly aggressive as far as action on the skin is concerned, such negative effects can also be avoided. In this connection, the positioning of the first absorption part 404 in the front portion 406 is especially advantageous as the tongue-shaped portion 404b can here be given such dimensions that the wetting zone is comprised in the first absorption part 404.

When an absorption body according to Fig. 4 is used in a diaper, the diaper may come to lie in close contact with the body of the wearer owing to pressure from the wearer. In this connection, a distance layer is suitably arranged between the liquid-permeable cover layer and the absorption body 404. The risk of liquid taken up in the free space 429 being pressed back so that what is known as rewetting occurs is then reduced.

Although the invention has been described above in connection with diapers, it is of course possible to apply the invention to absorbent articles such as incontinence shields, sanitary towels and panty liners as well.

## Claims

1. An absorbent article with a longitudinal direction and a transverse direction and comprising an absorption body which is enclosed in a cover and comprises at least one absorption layer, the cover having a liquid-permeable surface (102) and a liquid-impermeable surface (103), the absorption body comprises a second absorption part (105, 305, 405) which is separate from a first absorption part (104, 304, 404), **characterized in that**
the second absorption part (105, 305, 405) is displaced in the longitudinal direction from the first absorption part (104, 304, 404);
the first absorption part (104, 304, 404) has a tongue-shaped portion (104b, 304b, 404b) intended to be arranged in between two legs (105b, c; 305b, c; 405b, c) extending essentially in the longitudinal direction on the second absorption part (105, 305, 405) so that fold indications (126a, b; 326a, b; 426a, b) are formed in the absorption body between the tongue-shaped portion (104b, 304b, 404b) of the first absorption part (104, 304, 404) and at least each leg (105b, c; 305b, c; 405b, c) of the second absorption part (105, 305, 405);
the first absorption part (104, 304, 404) extends from the tongue-shaped portion (104b, 304b, 404b) in the longitudinal direction and in the transverse direction so that a large free surface (104a, 304a, 404a) is formed, which large free surface (104a, 304a, 404a) is considerably larger than the surface of the tongue-shaped portion (104b, 304b, 404b);
the second absorption part (105, 305, 405) extends from its legs (105b, c; 305b, c; 405b, c) in the longitudinal direction and in the transverse direction so that a large free surface (1 05a, 305a, 405a) is formed;
the second absorption part (105, 305, 405) has a tooth-like appearance; and
one of the first and second absorption parts (104, 304, 404, 105, 305, 405) forms a rear absorption part located in the rear end portion (107, 307, 407) of the article, which is intended to face backwards on the wearer during use, and the other of the first and second absorption parts (104, 304, 404, 105, 305, 405) forms a front absorption part located in the front end portion (106, 306, 406) of the article, which is intended to face forwards on the wearer during use.

2. An absorbent article according to Claim 1, the article having two end portions (106, 306, 406; 107, 307, 407) and a crotch portion (108, 308, 408) which is located between the end portions (106, 306, 406; 107, 307, 407) and has a smaller extent in the transverse direction than the end portions (106, 306, 406; 107, 307, 407), and the fold indications (126a, b; 326a, b; 426a, b) in the absorption body being arranged at least mainly in the crotch portion (108, 308, 408).

3. An absorbent article according to Claim 1 or 2, the two legs (105b, c; 305b, c; 405b, c) extending in the longitudinal direction on the second absorption part (105, 305, 405) having a length, s, of 3-30 cm, preferably 5-25 cm, and most preferably 7-20 cm.

4. An absorbent article according to any one of the preceding claims, the tongue-shaped portion (104b, 304b, 404b) of the first absorption part (104, 304, 404) having a length, t, of 3-30 cm, preferably 5-25 cm, and most preferably 7-20 cm.

5. An absorbent article according to any one of the preceding claims, there being a distance of 0-20 mm, preferably 2-15 mm, and most preferably 3-10 mm, between the tongue-shaped portion (104b, 304b, 404b) of the first absorption part (104, 304, 404) and each leg (105b, c; 305b, c; 405b, c) of the second absorption part (105, 305, 405) at least at the outer parts of the legs (1 05b, c; 305b, c; 405b, c).

6. An absorbent article according to any one of the preceding claims, the first absorption part (104, 304, 404) being locked in a predetermined position in relation to the second absorption part (105, 305, 405) by a stabilizing means.

7. An absorbent article according to Claim 6, the stabilizing means being a stabilizing layer which is connected firmly to the first absorption part (104, 304, 404) and the second absorption part (105, 305, 405).

8. An absorbent article according to Claim 7, the stabilizing layer constituting a distance layer between the first absorption part (104, 304, 404) and the second absorption part (105, 305, 405) and the liquid-permeable surface (102) of the cover, or the stabilizing layer consisting of the liquid-permeable surface (102) of the cover and/or of the liquid-impermeable surface (103) of the cover, or both cover layers (102, 103).

9. An absorbent article according to any of the preceding claims, the front absorption part comprising absorbent material intended for taking up urine and/or other viscous liquids such as menstrual fluids and discharges.

10. An absorbent article according to any of the preceding claims, the rear absorption part comprising absorbent material intended for taking up faeces and/or other viscous liquids such as menstrual fluids and discharges.

11. An absorbent article according to any one of the preceding claims, an inner edge of the legs (105b, c; 305b, c; 405b, c) of the second absorption part (105, 305, 405) coming in towards a central line so as thus to form a U-shaped edge which runs essentially edge to edge with the outer edge of the tongue-shaped portion (104b, 304b, 404b) of the first absorption part (104, 304, 404).

12. An absorbent article according to any one of the preceding claims, there being a distance between the U-shaped edge of the second absorption part (105, 305, 405) and the tongue-shaped portion (104b, 304b, 404b) of the first absorption part (104, 304, 404) of 1-20 mm, preferably 2-15 mm, and most preferably 3-10 mm, along the whole tongue-shaped portion (104b, 304b, 404b).

13. An absorbent article according to any one of Claims 1-10, the tongue-shaped portion (104b, 304b, 404b) of the first absorption part (104, 304, 404) overlapping at least a part of the second absorption part (105, 305, 405).

14. An absorbent article according to Claim 13, the tongue-shaped portion (104b, 304b, 404b) of the first absorption part (104, 304, 404) overlapping a part of the second absorption part (105, 305, 405) so that a double layer is formed in at least an area corresponding to the wetting zone of the absorbent article.

15. A method for producing an absorption body for use in an absorbent article according to any one of claims 1- 14, **characterized in that**
the absorption body is produced by a first absorption part (104, 304, 404) being shaped with a tongue-shaped portion (104b, 304b, 404b) and a second absorption part (105, 305, 405) being shaped with two legs (105b, c; 305b, c; 405b, c) extending in the longitudinal direction;
the first absorption part (104, 304, 404) is positioned separately and displaced in the longitudinal direction adjacent to the second absorption part (105, 305, 405) so that the tongue-shaped portion (104b, 304b, 404b) of the first absorption part (104, 304, 404) is arranged completely or partly in between the two legs (105b, c; 305b, c; 405b, c) extending essentially in the longitudinal direction on the second absorption part (105, 305, 405), fold indications (126a, b; 326a, b; 426a, b) then being formed in the absorption body between the tongue-shaped portion (104b, 304b, 404b) of the first absorption part (104, 304, 404) and at least each leg (105b, c; 305b, c; 405b, c) of the second absorption part (105, 305, 405);
the first absorption part (104, 304, 404) extends from the tongue-shaped portion (104b, 304b, 404b) in the longitudinal direction and in the transverse direction so that a large free surface (104a, 304a, 404a) is formed, which large free surface (104a, 304a, 404a) is considerably larger than the surface of the tongue-shaped portion (104b, 304b, 404b);
the second absorption part (105, 305, 405) extends from its legs (105b, c; 305b, c; 405b, c) in the longitudinal direction and in the transverse direction so that a large free surface (1 05a, 305a, 405a) is formed;
the second absorption part (105, 305, 405) has a tooth-like appearance; and
one of the first and second absorption parts (104, 304, 404, 105, 305, 405) forms a rear absorption part located in the rear end portion (107, 307, 407) of the article, which is intended to face backwards on the wearer during use, and the other of the first and second absorption parts (104, 304, 404, 105, 305, 405) forms a front absorption part located in the front end portion (106, 306, 406) of the article, which is intended to face forwards on the wearer during use.

16. Method according to Claim 15, at least a part of the inner edge of the legs (105b, c; 305b, c; 405b, c) of the second absorption part (105, 305, 405) being positioned edge to edge with the outer edge of the tongue-shaped portion (104b, 304b, 404b).

17. Method according to Claim 15, the first absorption part (104, 304, 404) being applied to the second absorption part (105, 305, 405), with which the tongue-shaped portion (104b, 304b, 404b) of the first absorption part (104, 304, 404) overlaps at least a part of the second absorption part (105, 305, 405).

18. Method according to any one of Claims 15-17, the shape of the absorption body after positioning of the first absorption part (104, 304, 404) and the second absorption part (105, 305, 405) in relation to one another being fixed by joining the absorption body together with a stabilizing layer.

19. Method according to any one of Claims 15-17, the shape of the absorption body after positioning of the first absorption part (104, 304, 404) and the second absorption part (105, 305, 405) in relation to one another being fixed by activating a bonding means included in the absorption body to a layer included in the absorbent article.

20. Method according to Claim 19, the bonding means comprising thermoplastic fibres, and the shape of the absorption body being stabilized by heating and subsequent cooling of the absorption body.

## Patentansprüche

1. Absorbierender Artikel mit einer Längsrichtung und einer Querrichtung umfassend einen Absorptionskörper, welcher in einer Abdeckung eingeschlossen ist und mindestens eine Absorptionsschicht umfasst, wobei die Abdeckung eine flüssigkeitsdurchlässige Oberfläche (102) und eine flüssigkeitsundurchlässige Oberfläche (103) aufweist, wobei der Absorptionskörper ein zweites Absorptionsteil (105, 305, 405) umfasst, welches von einem ersten Absorptionsteil (104, 304, 404) getrennt ist, **dadurch gekennzeichnet, dass** das zweite Absorptionsteil (105, 305, 405) von dem ersten Absorptionsteil (104, 304, 404) in der Längsrichtung versetzt ist;
das erste Absorptionsteil (104, 304, 404) ein zungenförmiges Teil (104b, 304b, 404b) aufweist, welches dazu vorgesehen ist, zwischen zwei Schenkeln (105b, c; 305b, c; 405b, c) angeordnet zu werden, und welches sich im Wesentlichen in der Längsrichtung auf dem zweiten Absorptionsteil (105, 305, 405) derart erstreckt, dass Falteindikationen (126a, b; 326a, b; 426a, b) in dem Absorptionskörper zwischen dem zungenförmigen Teil (104b, 304b, 404b) des ersten Absorptionsteils (104, 304, 404) und mindestens jedem Schenkel (105b, c; 305b, c; 405b, c) des zweiten Absorptionsteils (105, 305, 405) gebildet werden;
das erste Absorptionsteil (104, 304, 404) sich von dem zungenförmigen Teil (104b, 304b, 404b) in Längsrichtung und in Querrichtung derart erstreckt, dass eine große freie Oberfläche (104a, 304a, 404a) gebildet wird, welche große freie Oberfläche (104a, 304a, 404a) erheblich größer als die Oberfläche des zungenförmigen Teils (1 04b, 304b, 404b) ist;
das zweite Absorptionsteil (105, 305, 405) sich von seinen Schenkeln (105b, c; 305b, c; 405b, c) in Längsrichtung und in Querrichtung derart erstreckt, dass eine große freie Oberfläche (1 05a, 305a, 405a) gebildet wird;
das zweite Absorptionsteil (105, 305, 405) eine zahnartige Erscheinung aufweist; und
eines des ersten und des zweiten Absorptionsteils (104, 304, 404, 105, 305, 405) ein in dem hinteren Endabschnitt (107, 307, 407) des Artikels angeordnetes hinteres Absorptionsteil bildet, welches dazu vorgesehen ist, während des Gebrauchs an dem Benutzer nach hinten gerichtet zu sein, und das andere des ersten und des zweiten Absorptionsteils (104, 304, 404, 105, 305, 405) ein in dem vorderen Endabschnitt (106, 306, 406) angeordnetes vorderes Absorptionsteil bildet, welches dazu vorgesehen ist, während des Gebrauchs an dem Benutzer nach vorne gerichtet zu sein.

2. Absorbierender Artikel nach Anspruch 1, welcher zwei Endabschnitte (106, 306, 406; 107, 307, 407) und ein Schrittteil (108, 308, 408) aufweist, welches zwischen den Endabschnitten (106, 306, 406; 107, 307, 407) angeordnet ist und einen kleinere Erstreckung in Querrichtung als die Endabschnitte (106, 306, 406; 107, 307, 407) aufweist, und die Falteindikationen (126a, b; 326a, b; 426a, b) in dem Absorptionskörper mindestens hauptsächlich in dem Schrittteil (108, 308, 408) angeordnet sind.

3. Absorbierender Artikel nach Anspruch 1 oder 2, wobei die zwei, Schenkel (105b, c; 305b, c; 405b, c), die sich in Längsrichtung auf dem zweiten Absorptionsteil (105, 305, 405) erstrecken, eine Länge, s, von 3-30 cm, bevorzugt 5-25 cm, und eher bevorzugt 7-20 cm, aufweisen.

4. Absorbierender Artikel nach einem der vorgehenden Ansprüche, wobei das zungenförmige Teil (104b, 304b, 404b) des ersten Absorptionsteils (104, 304, 404) eine Länge, t, von 3-30 cm, bevorzugt 5-25 cm, und eher bevorzugt 7-20 cm, aufweist.

5. Absorbierender Artikel nach einem der vorgehenden Ansprüche, wobei ein Abstand von 0-20 mm, bevorzugt 2-15 mm, und eher bevorzugt 3-10 mm, zwischen dem zungenförmigen Teil (104b, 304b, 404b) des ersten Absorptionsteils (104, 304, 404) und jedem Schenkel (105b, c; 305b, c; 405b, c) des zweiten Absorptionsteils (105, 305, 405) zumindest an den äußeren Teilen der Schenkel (105b, c; 305b, c; 405b, c) besteht.

6. Absorbierender Artikel nach einem der vorgehenden Ansprüche, wobei das erste Absorptionsteil (104, 304, 404) in einer vorgegebenen Position relativ zum zweiten Absorptionsteil (105, 305, 405) mittels eines Stabilisierungsmittels verriegelt ist.

7. absorbierender Artikel nach Anspruch 6, wobei das Stabilisierungsmittel eine Stabilisierungsschicht ist, welche mit dem ersten Absorptionsteil (104, 304, 404) und dem zweiten Absorptionsteil (105, 305, 405) fest verbunden ist.

8. Absorbierender Artikel nach Anspruch 7, wobei die Stabilisierungsschicht eine Abstandsschicht zwischen dem ersten Absorptionsteil (104, 304, 404) und dem zweiten Absorptionsteil (105, 305, 405) und der flüssigkeitsdurchlässigen Oberfläche (102) der Abdeckung bildet, oder die Stabilisierungsschicht aus der flüssigkeitsdurchlässigen Oberfläche (102) der Abdeckung und/oder aus der flüssigkeitsundurchlässigen Oberfläche (103) der Abdeckung oder beider Abdeckungsschichten (102, 103) besteht.

9. Absorbierender Artikel nach einem der vorgehenden Ansprüche, wobei das vordere Absorptionsteil absorbierendes Material umfasst, welches zur Aufnahme von Urin und/oder anderen viskosen Flüssigkeiten, wie beispielsweise Menstruationsflüssigkeiten und Ausflüssen, vorgesehen ist.

10. Absorbierender Artikel nach einem der vorgehenden Ansprüche, wobei das hintere Absorptionsteil absorbierendes Material umfasst, welches zur Aufnahme von Fäzes und/oder anderen viskosen Flüssigkeiten, wie beispielsweise Menstruationsflüssigkeiten und Ausflüssen, vorgesehen ist.

11. Absorbierender Artikel nach einem der vorgehenden Ansprüche, wobei ein Innenrand der Schenkel (105b, c; 305b, c; 405b, c) des zweiten Absorptionsteils (105, 305, 405) auf eine Mittellinie zu verläuft, um somit einen U-förmigen Rand zu bilden, welcher im Wesentlichen Kante zu Kante mit dem Außenrand des zungenförmigen Teils (1 04b, 304b, 404b) des ersten Absorptionsteils (104, 304, 404) verlaüft.

12. Absorbierender Artikel nach einem der vorgehenden Ansprüche, wobei ein Abstand zwischen dem U-förmigen Rand des zweiten Absorptionsteils (105, 305, 405) und dem zungenförmigen Teil (104b, 304b, 404b) des ersten Absorptionsteils (104, 304, 404) von 1-20 mm, bevorzugt 2-15 mm, und eher bevorzugt 3-10 mm, entlang von dem gesamten zungenförmigen Teil (104b, 304b, 404b) besteht.

13. Absorbierender Artikel nach einem der Ansprüche 1-10, wobei das zungenförmige Teil (104b, 304b, 404b) des ersten Absorptionsteils (104, 304, 404) mindestens einen Teil des zweiten Absorptionsteils (105, 305, 405) überlappt.

14. Absorbierender Artikel nach Anspruch 13, wobei das zungenförmige Teil (104b, 304b, 404b) des ersten Absorptionsteils (104, 304, 404) einen Teil des zweiten Absorptionsteils (105, 305, 405) derart überlappt, dass eine Doppelschicht in zumindest einem dem Befeuchtungsbereich des absorbierenden Artikels entsprechenden Bereich gebildet wird.

15. Verfahren zur Herstellung eines Absorptionskörpers zur Verwendung in einem absorbierenden Artikel nach einem der Ansprüche 1-14, **dadurch gekennzeichnet, dass**
der Absorptionskörper durch mindestens ein erstes Absorptionsteils (104, 304, 404) hergestellt ist, welches mit einem zungenförmigen Teil (104b, 304b, 404b) gestaltet ist, und ein zweites Absorptionsteil (105, 305, 405) mit zwei in Längsrichtung sich erstreckenden Schenkeln (105b, c; 305b, c; 405b, c) gestaltet ist;
das erste Absorptionsteil (104, 304, 404) gesondert angeordnet ist und in Längsrichtung an das zweite Absorptionsteil (105, 305, 405) angrenzend derart versetzt ist, dass das zungenförmige Teil (104b, 304b, 404b) des ersten Absorptionsteils (104, 304, 404) völlig oder teilweise zwischen den zwei Schenkeln (105b, c; 305b, c; 405b, c) angeordnet ist, welche sich im Wesentlichen in Längsrichtung auf dem zweiten Absorptionsteil (105, 305, 405) erstrecken, wobei Falteindikationen (126a, b; 326a, b; 426a, b) dann in dem Absorptionskörper zwischen dem zungenförmigen Teil (104b, 304b, 404b) des ersten Absorptionsteils (104, 304, 404) und mindestens jedem Schenkel (105b, c; 305b, c; 405b, c) des zweiten Absorptionsteils (105, 305, 405) gebildet sind;
das erste Absorptionsteil (104, 304, 404) sich von dem zungenförmigen Teil (104b, 304b, 404b) in Längsrichtung und in Querrichtung derart erstreckt, dass eine große freie Oberfläche (104a, 304a, 404a) gebildet wird, welche große freie Oberfläche (104a, 304a, 404a) erheblich größer als die Oberfläche des zungenförmigen Teils (1 04b, 304b, 404b) ist;
das zweite Absorptionsteil (105, 305, 405) sich von seinen Schenkeln (105b, c; 305b, c; 405b, c) in Längsrichtung und in Querrichtung derart erstreckt, dass eine große freie Oberfläche (1 05a, 305a, 405a) gebildet wird;
das zweite Absorptionsteil (105, 305, 405) eine zahnartige Erscheinung aufweist; und
eines des ersten und des zweiten Absorptionsteils (104, 304, 404, 105, 305, 405) ein in dem hinteren Endabschnitt (107, 307, 407) des Artikels angeordnetes hinteres Absorptionsteil bildet, welches dazu vorgesehen ist, während des Gebrauchs an dem Benutzer nach hinten gerichtet zu sein, und das andere des ersten und des zweiten Absorptionsteils (104, 304, 404, 105, 305, 405) ein in dem vorderen Endabschnitt (106, 306, 406) angeordnetes vorderes Absorptionsteil bildet, welches dazu vorgesehen ist, während des Gebrauchs an dem Benutzer nach vorne gerichtet zu sein.

16. Verfahren nach Anspruch 15, wobei mindestens ein Teil des Innenrandes der Schenkel (105b, c; 305b, c; 405b, c) des zweiten Absorptionsteils (105, 305, 405) Kante zu Kante mit dem Außenrand des zungenförmigen Teils (104b, 304b, 404b) angeordnet wird.

17. Verfahren nach Anspruch 15, wobei das erste Absorptionsteil (104, 304, 404) auf das zweite Absorptionsteil (105, 305, 405) aufgetragen ist, mit welchem das zungenförmige Teil (104b, 304b, 404b) des ersten Absorptionsteils (104, 304, 404) mindestens einen Teil des zweiten Absorptionsteils (105, 305, 405) überlappt.

18. Verfahren nach einem der Ansprüche 15-17, wobei die Form des Absorptionsteils nach Positionierung des ersten Absorptionsteils (104, 304, 404) und des zweiten Absorptionsteils (105, 305, 405) relativ zueinander dadurch fixiert wird, dass der Absorptionskörper mit einer Stabilisierungschicht verbunden wird.

19. Verfahren nach einem der Ansprüche 15-17, wobei die Form des Absorptionskörpers nach Positionierung des ersten Absorptionsteils (104, 304, 404) und des zweiten Absorptionsteils (105, 305, 405) relativ zueinander dadurch fixiert wird, dass ein in dem Absorptionskörper eingeschlossenes Verbindungsmittel mit einer in dem absorbierenden Artikel eingeschlossenen Schicht aktiviert wird.

20. Verfahren nach Anspruch 19, wobei das Verbindungsmittel thermoplastische Fasern umfasst, und die Form des Absorptionskörpers durch Erhitzen und nachfolgende Kühlung des Absorptionskörpers stabilisiert wird.

## Revendications

1. Article absorbant avec une direction longitudinale et une direction transversale et comprenant un corps absorbant qui est enfermé dans un couvercle et comprend au moins une couche d'absorption, le couvercle comportant une surface perméable aux liquides (102) et une surface imperméable aux liquides (103), le corps absorbant comprend une deuxième partie d'absorption (105, 305, 405) qui est séparée de la première partie d'absorption (104, 304, 404),
**caractérisé en ce que**
la deuxième partie d'absorption (105, 305, 405) est déplacée dans la direction longitudinale à partir de la première partie d'absorption (104, 304, 404); la première partie d'absorption (104, 304, 404) présente une portion en forme de languette (104b, 304b, 404b) destinée à être disposée entre deux branches (105b, c; 305b, c; 405b, c) s'étendant essentiellement dans la direction longitudinale sur la deuxième partie d'absorption (105, 305, 405) si bien que des indications de pliage (126a, b; 326a, b; 426a, b) sont formées dans le corps absorbant entre la portion en forme de languette (104b, 304b, 404b) de la première partie d'absorption (104, 304, 404) et au moins chaque branche (105b, c; 305b, c; 405b, c) de la deuxième partie d'absorption (105, 305, 405);
la première partie d'absorption (104, 304, 404) s'étend à partir de la portion en forme de languette (104b, 304b, 404b) dans la direction longitudinale et dans la direction transversale si bien qu'une grande surface libre (104a, 304a, 404a) est formée, ladite grande surface libre (104a, 304a, 404a) étant considérablement supérieure à la surface de la portion en forme de languette (104b, 304b, 404b);
la deuxième partie d'absorption (105, 305, 405) s'étend à partir de ses branches (105b, c; 305b, c; 405b, c) dans la direction longitudinale et dans la direction transversale si bien qu'une grande surface libre (105a, 305a, 405a) est formée;
la deuxième partie d'absorption (105, 305, 405) présente une apparence semblant à dent; et
l'une des première et deuxième parties d'absorption (104, 304, 404, 105, 305, 405) forme une partie d'absorption arrière située dans la partie d'extrémité arrière (107, 307, 407) de l'article qui est destinée à être tournée vers l'arrière sur l'utilisateur pendant l'utilisation, et l'autre des première et deuxième parties d'absorption (104, 304, 404, 105, 305, 405) forme une partie d'absorption avant située dans la partie d'extrémité avant (106, 306, 406) de l'article qui est destinée à être tournée vers l'avant sur l'utilisateur pendant l'utilisation.

2. Article absorbant selon la revendication 1, l'article présentant deux parties d'extrémité (106, 306, 406; 107, 307, 407) et une partie d'entre-jambes (108, 308, 408) qui est située entre les parties d'extrémité (106, 306, 406; 107, 307, 407) et présente une étendue dans la direction transversale qui est inférieure à celle des parties d'extrémité (106, 306, 406; 107, 307, 407), et les indications de pliage (126a, b; 326a, b; 426a, b) dans le corps absorbant étant arrangées au moins essentiellement dans la partie d'entre-jambes (108, 308, 408).

3. Article absorbant selon la revendication 1 ou 2, les deux branches (105b, c; 305b, c; 405b, c) s'étendant dans la direction longitudinale sur la deuxième partie d'absorption (105, 305, 405) ayant une longueur, s, comprise entre 3 et 30 cm, préférablement entre 5 et 25 cm, et le plus préférablement entre 7 et 20 cm.

4. Article absorbant selon l'une quelconque des revendications précédentes, la portion en forme de languette (104b, 304b, 404b) de la première partie d'absorption (104, 304, 404) ayant une longueur, t, comprise entre 3 et 30 cm, préférablement entre 5 et 25 cm, et le plus préférablement 7 et 20 cm.

5. Article absorbant selon l'une quelconque des revendications précédentes, une distance comprise entre 0 et 20 mm, préférablement entre 2 et 15 mm, et le plus préférablement entre 3 et 10 mm, étant pourvue entre la portion en forme de languette (104b, 304b, 404b) de la première partie d'absorption (104, 304, 404) et chaque branche (105b, c; 305b, c; 405b, c) de la deuxième partie d'absorption (105, 305, 405) au moins aux portions extérieures des branches (1 05b, c; 305b, c; 405b, c).

6. Article absorbant selon l'une quelconque des revendications précédentes, la première partie d'absorption (104, 304, 404) étant bloquée dans une position prédéterminée par rapport à la deuxième partie d'absorption (105, 305, 405) par un moyen de stabilisation.

7. Article absorbant selon la revendication 6, le moyen de stabilisation étant une couche de stabilisation qui est reliée solidement à la première partie d'absorption (104, 304, 404) et la deuxième partie d'absorption (105, 305, 405).

8. Article absorbant selon la revendication 7, la couche de stabilisation constituant une couche de distance entre la première partie d'absorption (104, 304, 404) et la deuxième partie d'absorption (105, 305, 405) et la surface perméable aux liquides (102) du couvercle, ou la couche de stabilisation composée de la surface perméable aux liquides (102) du couvercle et/ou de la surface imperméable aux liquides (103) du couvercle, ou des deux couches du couvercle (102, 103).

9. Article absorbant selon l'une quelconque des revendications précédentes, la partie d'absorption avant comprenant un matériau absorbant destiné à absorber l'urine et/ou d'autres liquides visqueux tels que les fluides menstruels et des évacuations.

10. Article absorbant selon l'une quelconque des revendications précédentes, la partie d'absorption arrière comprenant un matériau absorbant destiné à absorber des fèces et/ou d'autres liquides visqueux tels que les fluides menstruels et des évacuations.

11. Article absorbant selon l'une quelconque des revendications précédentes, un bord intérieur des branches (1 05b, c; 305b, c; 405b, c) de la deuxième partie d'absorption (105, 305, 405) entrant en direction d'une ligne centrale pour ainsi former un bord en U qui s'étend essentiellement bord à bord avec le bord extérieur de la portion en forme de languette (104b, 304b, 404b) de la première partie d'absorption (104, 304, 404).

12. Article absorbant selon l'une quelconque des revendications précédentes, une distance étant pourvue entre le bord en U de la deuxième partie d'absorption (105, 305, 405) et la portion en forme de languette (104b, 304b, 404b) de la première partie d'absorption (104, 304, 404) comprise entre 1 et 20 mm, préférablement 2 et 15 mm, et le plus préférablement 3 et 10 mm, le long de la portion en forme de languette (1 04b, 304b, 404b).

13. Article absorbant selon l'une quelconque des revendications 1 à 10, la portion en forme de languette (104b, 304b, 404b) de la première partie d'absorption (104, 304, 404) chevauchant au moins une portion de la deuxième partie d'absorption (105, 305, 405).

14. Article absorbant selon la revendication 13, la portion en forme de languette (104b, 304b, 404b) de la première partie d'absorption (104, 304, 404) chevauchant une portion de la deuxième partie d'absorption (105, 305, 405) si bien qu'une double couche est formée dans au moins une zone correspondant à la zone de mouillage de l'article absorbant.

15. Procédé de fabrication d'un corps absorbant pour une utilisation dans un article absorbant selon l'une quelconque des revendications 1 à 14,
**caractérisé en ce que**
le corps absorbant est produit par une première partie d'absorption (104, 304, 404) étant formée avec une portion en forme de languette (104b, 304b, 404b) et une deuxième partie d'absorption (105, 305, 405) étant formée avec deux branches (105b, c; 305b, c; 405b, c) s'étendant dans la direction longitudinale;
la première partie d'absorption (104, 304, 404) est disposée séparément et déplacée dans la direction longitudinale adjacente à la deuxième partie d'absorption (105, 305, 405) si bien que la portion en forme de languette (104b, 304b, 404b) de la première partie d'absorption (104, 304, 404) est arrangée totalement ou partiellement entre les deux branches (105b, c; 305b, c; 405b, c) s'étendant essentiellement dans la direction longitudinale sur la deuxième partie d'absorption (105, 305, 405), des indications de pliage (126a, b; 326a, b; 426a, b) étant alors formées dans le corps absorbant entre la portion en forme de languette (104b, 304b, 404b) de la première partie d'absorption (104, 304, 404) et au moins chaque branche (1 05b, c; 305b, c; 405b, c) de la deuxième partie d'absorption (105, 305, 405);
la première partie d'absorption (104, 304, 404) s'étend à partir de la portion en forme de languette (104b, 304b, 404b) dans la direction longitudinale et dans la direction transversale si bien qu'une grande surface libre (104a, 304a, 404a) est formée, ladite grande surface libre (104a, 304a, 404a) étant considérablement supérieure à la surface de la portion en forme de languette (104b, 304b, 404b);
la deuxième partie d'absorption (105, 305, 405) s'étend à partir de ses branches (105b, c; 305b, c; 405b, c) dans la direction longitudinale et dans la direction transversale si bien qu'une grande surface libre (105a, 305a, 405a) est formée;
la deuxième partie d'absorption (105, 305, 405) présente une apparence semblant à dent; et
l'une des première et deuxième parties d'absorption (104, 304, 404, 105, 305, 405) forme une partie d'absorption arrière située dans la partie d'extrémité arrière (107, 307, 407) de l'article qui est destinée à être tournée vers l'arrière sur l'utilisateur pendant l'utilisation, et l'autre des première et deuxième parties d'absorption (104, 304, 404, 105, 305, 405) forme une partie d'absorption avant située dans la partie d'extrémité avant (106, 306, 406) de l'article qui est destinée à être tournée vers l'avant sur l'utilisateur pendant l'utilisation.

16. Procédé selon la revendication 15, au moins une portion du bord intérieur des branches (105b, c; 305b, c; 405b, c) de la deuxième partie d'absorption (105, 305, 405) étant positionnée bord à bord avec le bord extérieur de la portion en forme de languette (1 04b, 304b, 404b).

17. Procédé selon la revendication 15, la première partie d'absorption (104, 304, 404) étant appliquée à la deuxième partie d'absorption (105, 305, 405), avec laquelle la portion en forme de languette (104b, 304b, 404b) de la première partie d'absorption (104, 304, 404) chevauche au moins une portion de la deuxième partie d'absorption (105, 305, 405).

18. Procédé selon l'une quelconque des revendications 15 à 17, la forme du corps absorbant après le positionnement de la première partie d'absorption (104, 304, 404) et de la deuxième partie d'absorption (105, 305, 405) l'une par rapport à l'autre étant fixée en joignant le corps absorbant avec une couche de stabilisation.

19. Procédé selon l'une quelconque des revendications 15 à 17, la forme du corps absorbant après le positionnement de la première partie d'absorption (104, 304, 404) et de la deuxième partie d'absorption (105, 305, 405) l'une par rapport à l'autre étant fixée en activant un moyen de liaison compris dans le corps absorbant à une couche comprise dans l'article absorbant.

20. Procédé selon la revendication 19, le moyen de liaison comprenant des fibres thermoplastiques, et la forme du corps absorbant étant stabilisée par un chauffage et un refroidissement subséquent du corps absorbant.
